# FASCICULE DE BREVET EUROPEEN

(11) **EP 3 068 496 B1**
(45) Date de publication et mention de la délivrance du brevet: **08.11.2017**
(21) Numéro de dépôt: 14798808.3
(22) Date de dépôt: 12.11.2014
(51) Int. Cl.: A61Q 19/00, A61Q 19/08, A61K 8/92, A61K 8/97, A61K 36/906, A61K 36/88

(54) **COMPOSITION HUILEUSE A BASE D'EXTRAITS LIPOPHILES DE ROSE DE PORCELAINE ET DE JACINTHE D'EAU**
ÖLIGE ZUSAMMENSETZUNG AUF DER BASIS VON LIPOPHILEN EXTRAKTEN AUS FACKELINGWER UND WASSERHYAZINTHE
OILY COMPOSITION BASED ON LIPOPHILIC EXTRACTS OF TORCH GINGER AND WATER HYACINTH

(30) Priorité: 12.11.2013 FR 1361005
(43) Date de publication de la demande: 21.09.2016
(73) Titulaire: Société De Recherche Cosmétique S.à.r.L., 2314 Luxembourg (LU)
(72) Inventeur: LECONTE, Nadine, F-92600 Asnieres sur Seine (FR); ROSSIGNOL-CASTERA, Anne, F-34160 Restinclieres (FR)
(74) Mandataire: Goulard, Sophie
(86) Numéro de dépôt international: PCT/EP2014/074372
(87) Numéro de publication internationale: WO 2015/071307

(56) Documents cités:
- WO-A1-01/87319
- FR-A1- 2 809 007
- FR-A1- 2 943 684
- JP-A- 2004 123 660
- ERIC W.C. CHAN ET AL: "Phytochemistry and Pharmacological Properties of Etlingera elatior: A Review", PHARMACOGNOSY JOURNAL, vol. 3, no. 22, 1 juin 2011 (2011-06-01), pages 6-10, XP055125730, ISSN: 0975-3575, DOI: 10.5530/pj.2011.22.2
- M.M. JEEVANI OSADEE WIJEKOON ET AL: "Effect of extraction solvents on the phenolic compounds and antioxidant activities of bunga kantan (Etlingera elatior Jack.) inflorescence", JOURNAL OF FOOD COMPOSITION AND ANALYSIS, vol. 24, no. 4-5, 1 juin 2011 (2011-06-01) , pages 615-619, XP055126050, ISSN: 0889-1575, DOI: 10.1016/j.jfca.2010.09.018

## Description

La présente invention se rapporte au domaine technique général des produits cosmétiques à usage topique, utilisables notamment sur la peau.

Plus précisément, la présente invention est relative à une nouvelle composition huileuse à base d'extraits lipophiles de rose de porcelaine et de jacinthe d'eau, aux compositions cosmétiques comprenant une telle composition huileuse, à un procédé de traitement cosmétique non thérapeutique de la peau mettant en oeuvre une telle composition cosmétique, ainsi qu'à l'utilisation non thérapeutique d'une composition cosmétique à application topique comprenant un extrait lipophile de rose de porcelaine et de jacinthe d'eau pour améliorer l'éclat de la peau.

La peau est un véritable organe comprenant plusieurs couches intégrées, allant de la couche superficielle, l'épiderme, jusqu'aux couches plus profondes, le derme et l'hypoderme, et chacune de ces couches possède des propriétés spécifiques permettant à l'ensemble de réagir et de s'adapter aux conditions de son environnement.

L'épiderme est principalement composé de kératinocytes (90% des cellules épidermiques) qui synthétisent la kératine, de mélanocytes (2 à 3% des cellules épidermiques) responsables de la pigmentation de la peau, et des cellules de Langerhans qui jouent un rôle immunologique. L'épiderme, dont l'épaisseur est variable selon les différentes parties du corps, constitue la couche externe de la peau et par conséquent il joue un rôle fondamental pour assurer la protection et le maintien d'une bonne trophicité. C'est pourquoi de nombreuses compositions ont été mises au point afin de le protéger, notamment des agressions de l'environnement.

Le derme est classiquement divisé en deux régions qui diffèrent par la composition et l'organisation de leur matrice extracellulaire respective :
- le derme papillaire, le plus superficiel, qui se trouve sous l'épiderme et forme les papilles dermiques entre les crêtes épidermiques. C'est un tissu conjonctif lâche constitué de fines fibrilles de collagène de type I et III, isolées et orientées le plus souvent perpendiculairement ou obliquement par rapport à la jonction dermo-épidermique. Des fibres élastiques oxytalanes également orientées perpendiculairement à l'épiderme forment des structures en forme de chandelles. Le plexus vasculaire sous les crêtes papillaires (*rete subpapillare*) délimite la limite inférieure du derme papillaire. Les capillaires qui s'étendent depuis le plexus sous-papillaire se projettent dans les papilles dermiques pour apporter les nutriments nécessaires à l'épiderme ;
- le derme réticulaire plus profond qui est un tissu conjonctif plus dense composée d'un entrecroisement de faisceaux de grosses fibres de collagène et de fibres élastiques présentant une orientation préférentiellement parallèle à la surface de la peau. Le derme réticulaire contient moins de collagène de type III que le derme papillaire. Un plexus vasculaire profond (*rete cutaneum*) et la transition entre un tissu fibreux et un tissu adipeux marque la limite inférieure du derme réticulaire.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

La notion d'éclat de la peau est très complexe, et d'origine multifactorielle. L'éclat de la peau peut être défini comme résultant du mélange pondéré des caractéristiques de la texture de la surface cutanée, de sa structure, de sa brillance, de sa microcirculation et de sa couleur.

La microcirculation est l'une des clefs de l'éclat de la peau, de sa vitalité et de sa couleur. C'est au niveau du derme papillaire que parviennent les terminaisons vasculaires, lesquelles jouent un rôle fondamental dans les échanges avec l'épiderme, en assurant des fonctions primordiales d'oxygénation, de nutrition, d'élimination, etc ... Grâce à la microcirculation, la peau « respire », elle renvoie un éclat rosé particulièrement vivace à certains moments, signe d'une pleine santé. Si la microcirculation ralentit, la peau pâlit, se ternit, non seulement pour des raisons purement colorielles liées à l'irrigation sanguine, mais également du fait d'un manque d'oxygénation qui accentue sa perte de fraicheur.

Les produits d'origine naturelle ou contenant des composés naturels sont par ailleurs de plus en plus appréciés par les consommateurs et consommatrices de produits cosmétiques. C'est pourquoi on trouve sur le marché des produits cosmétiques de plus en plus de produits contenant des substances naturelles ou d'origine naturelle.

De telles compositions contiennent classiquement au moins une substance anti-oxydante, en particulier des molécules complexes d'origine végétale telles que par exemple les polyphénols, les flavonoïdes, les anthocyanosides, les caroténoïdes, etc...

Parmi les flavonoïdes, on peut notamment mentionner la rutine qui est un diglycoside composé de quercétine et de rutinose (rhamnnose et glucose). La rutine est présente dans de nombreuses espèces végétales et est connue pour diminuer la perméabilité et la fragilité capillaire (Frericks CT et al., J Lab. Clin Med, 35(6): 933-939. 1950). Elle a déjà été utilisée en cosmétique notamment pour le traitement de la couperose. La quercétine (ou quercétol) est un antioxydant puissant dont l'utilisation à titre de piégeur de radicaux libres dans des compositions cosmétiques est également connue.

Ces molécules ne sont toutefois pas toujours assez stables dans le temps et perdent ainsi progressivement leur efficacité.

Il est donc difficile, à l'heure actuelle, d'obtenir des compositions cosmétiques contenant un extrait végétal d'origine naturelle et dont les propriétés restent stables dans le temps, alors même que le besoin semble important.

La rose de porcelaine (*Etlingera elatior*) est une fleur exotique appartenant à la famille des Zingiberaceae. Ce sont des plantes herbacées vivaces, parfois de grande taille, qui sont répandues du sous-continent indien aux îles du Pacifique, avec un maximum de variétés en Malaisie.

L'une des plus connues est la rose de porcelaine car elle a la plus grande activité anti-oxydante et antibactérienne des espèces étudiées. Plusieurs parties de la rose porcelaine sont intéressantes : les rhizomes, la tige, les feuilles et la fleur. Les propriétés antioxydantes des feuilles sont significativement plus grandes que celles des fleurs et des rhizomes.

A partir des feuilles de l'*E*. *elatior*, trois acides cafeoylquiniques (320 mg/100 g) (incluant l'acide chlorogénique : 294 mg/100 g) et trois flavonoïdes ont été isolés. Les flavonoïdes présents dans les feuilles sont principalement représentés par la quercétine (79 mg/100g), l'isoquercétine (117 mg/100 g) et, dans une moindre mesure, la catéchine. L'acide chlorogénique est l'ester de l'acide caféique et de l'acide quinique. Il a été établi que l'acide chlorogénique est un antioxydant naturel et a des applications en médecine, en alimentaire et en cosmétique (E.C.W. Chiang, Monash University, 2009, Monash University Theses ; E.W.C. Chan et al., Food Chemistry, 2007, 104).

La jacinthe d'eau (*Eichhornia crassipes*) est une plante aquatique de la famille des Pontederiaceae, originaire de la zone amazonienne, qui s'est développée dans diverses régions tropicales et subtropicales où sa croissance rapide, parfois de 2 à 5 m par jour, en fait une plante invasive dont les tiges forment des tapis flottants denses et dont les feuilles se tiennent sur les tiges, au-dessus de la surface de l'eau. Les fleurs, rassemblées en épis de 8 à 15 fleurs au sommet des tiges, comportent chacune six pétales de couleur bleu violacé à rose, le pétale du sommet étant jaune. Le fruit de la jacinthe d'eau forme une capsule pouvant contenir plus de 300 graines.

La rapidité de sa croissance fait de la jacinthe d'eau une plante aquatique invasive provoquant des effets néfastes sur les écosystèmes en limitant la lumière nécessaire à la vie aquatique. Elle a parfois été utilisée pour l'alimentation du bétail, pour la fabrication de meubles artisanaux en Asie et en Afrique, ou pour le traitement des eaux usées en raison de la capacité de ses racines à absorber les métaux lourds.

La jacinthe d'eau peut accumuler de nombreux composants polluants, et elle agit alors sur son environnement comme agent détoxifiant. Mais cette accumulation de polluants génère des dérivés actifs de l'oxygène dans les cellules de la jacinthe d'eau. L'antioxydant majeur des cellules, le glutathion sous forme réduite, va alors protéger les cellules des radicaux libres. La jacinthe d'eau possède donc une activité anti-oxydante et la quantité de glutathion présente dans ses feuilles a été estimée à 40 nmoles par gramme de plante sèche.

Divers travaux ont été réalisés pour tenter de valoriser la jacinthe d'eau et par exemple, la demande de brevet US 2005/214389 décrit un procédé d'extraction de β-carotène à partir d'*Eichhornia crassipes* dans un solvant organique après séchage et broyage de la plante. Une activité hypolipidémiante d'un extrait d'*Eichhornia crassipes* a été décrite dans le brevet CN 101337009. La demande de brevet WO 0187319 décrit l'utilisation d'extraits cellulaires de jacinthe d'eau dans des compositions cosmétiques comme agent anti-pollution ou de dépollution pour protéger la peau des effets nocifs des métaux lourds, tels que cadmium, nickel et plomb présents dans l'environnement. Cependant, la possibilité d'utiliser des extraits d'*Eichhornia crassipes,* ou jacinthe d'eau, pour améliorer l'éclat du teint n'a jamais été suggérée.

Les études effectuées par la Demanderesse ont montré qu'une composition huileuse contenant, un extrait lipophile de rose de porcelaine (*Etlingera elatior*) et un extrait lipophile de Jacinthe d'eau (*Eichhornia crassipes*), présente, en plus de propriétés anti-oxydantes puissantes, des propriétés d'activation de la microcirculation permettant d'augmenter la composante rosée de la peau et de diminuer les composantes jaunes et vertes conduisant ainsi à une amélioration significative de l'éclat du teint.

De plus, les essais de toxicologie effectués sur des volontaires ont montré que ces extraits n'ont aucun effet irritant ou allergisant pour la peau.

La présente invention a donc pour premier objet une composition huileuse, caractérisée en ce qu'elle comprend :
i) au moins une huile végétale V1 renfermant au moins un extrait lipophile de rose de porcelaine (*Etlingera elatior*) et
ii) au moins une huile végétale V2, renfermant au moins un extrait lipophile de jacinthe d'eau (*Eichhornia crassipes*) ;
lesdites huiles V1 et V2 (huiles vectrices) étant identiques ou différentes l'une de l'autre.

Les compositions cosmétiques à application topique comprenant une telle composition huileuse à titre d'ingrédients actifs constituent un autre objet de l'invention. Outre leurs excellentes propriétés anti-oxydantes utilisables en cosmétique pour les soins de la peau, plus particulièrement pour protéger la peau des agressions de l'environnement et pour prévenir les signes du vieillissement cutané, de telles compositions présentent également des propriétés d'activation de la microcirculation permettant d'augmenter la composante rosée de la peau et de diminuer les composantes jaunes et vertes conduisant ainsi à une amélioration significative de l'éclat du teint. Les propriétés anti-oxydantes et d'activation de la microcirculation des compositions cosmétiques incorporant la composition huileuse conforme à l'invention sont de plus particulièrement stables dans le temps.

L'invention a donc également pour autre objet, un procédé cosmétique non thérapeutique pour améliorer l'éclat de la peau, consistant à appliquer sur les zones de la peau concernées au moins une composition cosmétique telle que définie ci-dessus, c'est-à-dire une composition cosmétique topique contenant une quantité efficace de la composition huileuse conforme à l'invention.

Enfin, l'invention a pour objet l'utilisation non thérapeutique d'une composition cosmétique à application topique comprenant une composition huileuse telle que définie précédemment pour améliorer l'éclat de la peau.

Au sein de la composition huileuse conforme à l'invention, la quantité d'huile végétale V1 renfermant l'extrait lipophile de rose de porcelaine varie de préférence de 70 à 95 % en masse, et encore plus préférentiellement de 85 à 90 % en masse environ et la quantité d'huile végétale V2 renfermant l'extrait lipophile de jacinthe d'eau varie de préférence de 30 à 5 % en masse environ, et encore plus préférentiellement de 15 à 10 % en masse environ, lesdits pourcentages étant exprimés par rapport à la masse totale de la composition huileuse.

Selon une forme de réalisation tout particulièrement préférée de l'invention, la composition huileuse comprend environ 90 % en masse d'huile végétale V1 renfermant l'extrait lipophile de rose de porcelaine et environ 10 % en masse d'huile végétale V2 renfermant l'extrait lipophile de jacinthe d'eau.

L'extrait lipophile de rose de porcelaine comprend des molécules hydrophiles extraites de la plante, et notamment :
- acides chlorogéniques : de 200 à 400 mg/kg, de préférence de 200 à 300 mg/kg ;
- quercétine : de 50 à 200 mg/kg, de préférence de 50 à 100 mg/kg ;
- oleuropéine : de 300 à 600 mg/kg, de préférence de 500 à 600 mg/kg.

L'extrait lipophile de jacinthe d'eau comprend également des molécules hydrophiles, et notamment :
- acides chlorogéniques : de 400 à 600 mg/kg, de préférence de 500 à 600 mg/kg ;
- quercétine : de 100 à 200 mg/kg, de préférence de 150 à 200 mg/kg ;
- oleuropéine : de 700 à 900 mg/kg, de préférence de 800 à 900 mg/kg.

Les huiles végétales V1 et V2, identiques ou différentes l'une de l'autre, sont de préférence choisies parmi l'huile de colza, l'huile de jojoba, l'huile d'avocat, l'huile de rosier muscat, l'huile de noisette, l'huile de macadamia, l'huile d'argan, l'huile de tournesol, l'huile de cameline, l'huile de bourrache, huile d'amande douce, huile de carthame, huile de calophyllum et leurs mélanges.

Selon, une forme de réalisation préférée de l'invention, la composition huileuse comprend un mélange d'huile de colza (HC) et d'huile de jojoba (HJ), de préférence dans un rapport massique HC/HJ variant de 70/30 à 90/10, le rapport massique d'environ 90/10 étant tout particulièrement préféré.

Ainsi, selon une forme de réalisation encore plus préférée de l'invention, la composition huileuse comprend environ 90 % en masse d'huile de colza renfermant au moins un extrait lipophile de rose de porcelaine et environ 10 % en masse d'huile de jojoba renfermant au moins un extrait lipophile de jacinthe d'eau.

En ce qui concerne la rose de porcelaine, les études effectuées par la demanderesse ont montré que l'on peut utiliser plusieurs parties de la plante, aussi bien les rhizomes, la tige, les feuilles et la fleur, les propriétés antioxydantes des feuilles étant néanmoins significativement plus grandes que celles des fleurs et des rhizomes. L'espèce *Etlingera elatior* est facilement disponible et la conservation des plantes ne soulève généralement pas de difficulté technique.

En ce qui concerne la jacinthe d'eau, les études effectuées par la demanderesse ont montré que l'on peut utiliser toutes les parties de la plante, aussi bien les racines que les parties aériennes, notamment les écorces, les tiges, les feuilles, les fleurs, les fruits et les graines, mais on utilise de préférence les parties aériennes. L'espèce *Eichhornia crassipes* est facilement disponible et la conservation des plantes ne soulève généralement pas de difficulté technique non plus.

La composition huileuse conforme à l'invention peut être préparée selon le procédé d'extraction décrit dans la demande internationale WO 2010/112760 consistant, dans une première étape, à mélanger et imprégner la plante, préalablement séchée et broyée, dans au moins une huile végétale naturelle à une température supérieure à son point de fusion, puis à chauffer le mélange à haute température pendant une durée très courte, et à former une microdispersion à une température supérieure à la température de fusion de l'huile végétale. Toutes ces étapes se font de préférence à l'abri de l'air, plus particulièrement à l'abri de l'oxygène pour éviter toute réaction d'oxydation des extraits. On peut par exemple travailler sous atmosphère d'azote, dans un réacteur clos avec extraction continue de l'oxygène par un flux d'azote, ou encore sous vide.

Les huiles végétales naturelles utilisables dans ce procédé sont de préférence choisies parmi l'huile de jojoba, l'huile de colza, l'huile d'avocat, l'huile de rosier muscat, l'huile de noisette, l'huile de macadamia, l'huile d'argan, l'huile de tournesol, l'huile de cameline, l'huile de bourrache, huile d'amande douce, huile de carthame, huile de calophyllum et leurs mélanges.

Selon, une forme de réalisation préférée de l'invention, l'huile végétale est choisie parmi l'huile de jojoba, l'huile de colza et leurs mélanges.

Selon l'invention, le procédé d'extraction est de préférence appliqué à chacune des plantes séparément (rose de porcelaine et jacinthe d'eau), en utilisant respectivement de l'huile de colza pour réaliser l'extraction de la rose de porcelaine et de l'huile de jojoba pour réaliser l'extraction de la jacinthe, puis les extraits lipophiles ainsi obtenus sont ensuite réunis pour conduire à la composition huileuse conforme à l'invention dans les proportions désirées et telles que définies précédemment.

Les huiles végétales utilisées selon l'invention, et en particulier les huiles de jojoba et de colza, sont des produits disponibles dans le commerce.

L'huile de jojoba est en fait une cire naturelle liquide constituée à plus de 98 % d'esters aliphatiques insaturés à base d'acides gras et d'alcools gras mono-insaturés, majoritairement à 20 et 22 atomes de carbone et une double liaison, C₂₀ :1 et C₂₂ :1. C'est la présence de doubles liaisons qui explique que le point de fusion de cette cire soit en dessous de la température ambiante d'où son aspect liquide comme une huile. Sa composition est proche de celle du sébum naturel qui recouvre la peau, ce qui lui confère des propriétés nutritives et émollientes remarquables et une parfaite tolérance cutanée.

L'huile de colza a une composition particulièrement équilibrée (ratio de 2,5 entre Oméga 6 et Oméga 3 notamment). Elle contient naturellement des Oméga 9, Oméga 6 et Oméga 3. Elle est naturellement riche en vitamine E reconnue comme antioxydant naturel.

L'utilisation de ces huiles végétales comme solvant des extraits de rose de porcelaine et de jacinthe d'eau présente l'avantage d'être compatible avec les normes des produits certifiés Ecocert s'appliquant aux produits cosmétiques à la fois écologiques et biologiques.

La première étape du procédé ci-dessus, consistant à imprégner la plante, de préférence broyée, se déroule à température ambiante, ou à chaud, à une température supérieure à celle de fusion des huiles utilisées. On travaille de préférence à température ambiante, de l'ordre de 25°C, les huiles végétales, et en particulier les huiles de jojoba et de colza, étant liquides à cette température.

Selon une forme de réalisation préférée, le broyage de la plante est effectué à une température d'environ -80°C, jusqu'à obtention d'une poudre présentant une granulométrie moyenne inférieure à 100 µm environ.

La deuxième étape comporte un chauffage à une température élevée, par exemple de l'ordre de 140 à 170°C, pendant une courte durée, généralement inférieure à 5 minutes. Le temps de montée en température doit aussi être très court. La technique du chauffage par micro-ondes est bien adaptée à ces conditions. La technique des micro-ondes facilite aussi la troisième étape de microdispersion des particules dans la ou les huiles végétales utilisées, avec rupture des membranes cellulaires. Cette troisième étape peut aussi comprendre un traitement par cavitation ultrasonore, de préférence dans un réacteur fermé équipé d'un générateur d'ultrasons à basse fréquence. Cette troisième étape peut éventuellement être réalisée simultanément avec les deux autres, l'ensemble des étapes, ou chacune d'elles, pouvant être réalisé plusieurs fois si nécessaire.

Selon une forme de réalisation préférée de l'invention, l'extraction de la rose de porcelaine est réalisée en utilisant de l'huile de colza et l'extraction de la jacinthe d'eau est réalisée en utilisant de l'huile de jojoba.

A la fin de l'extraction, les extraits lipophiles sont de préférence filtrés sur un filtre ayant une porosité inférieure à environ 5 µm, de façon à obtenir des extraits lipophiles limpides.

Les caractéristiques à température ambiante des extraits lipophiles obtenus à partir de la rose de porcelaine et de la jacinthe d'eau, ainsi que de la composition huileuse résultant de leur mélange dans un rapport volumique extrait lipophile de rose de porcelaine/ extrait lipophile de jacinthe d'eau 90/10 sont reportées dans le tableau 1 ci-après :

**Tableau 1**

| | | Extrait lipophile de rose de porcelaine | Extrait lipophile de jacinthe d'eau | Composition huileuse (%/% volume) |
|---|---|---|---|---|
| Huile Vectrice | | Huile de colza | Huile de Jojoba | Huile de colza 90/ huile de jojoba 10 |
| Aspect général | | Homogène, fluide | Homogène, fluide | Homogène, fluide |
| Couleur | | Vert clair | Vert | Vert |
| Limpidité | | Limpide | Limpide | Limpide |
| Indice de peroxyde (meqO₂/kg (NF EN ISO 3960) | | 1,67 | 0,41 | 2,46 |
| Acidité oléique (%) (NF EN ISO 660) | | 1,14 | 0,89 | 0,84 |
| Teneur en eau et en volatils (%) (NF EN ISO 662) | | 0,02 | 0,02 | 0,01 |
| Teneurs en phénols totaux (dosage par HPLC) (mg/kg) | Classe des acides phénoliques (éq. Acide chlorogénique) | 243 | 506 | 442 |
| | Classe des flavonoïdes (éq. quercétine) | 89 | 185 | 162 |

De plus, la technique utilisée permet d'obtenir une composition huileuse bien plus concentrée en principes actifs qu'un simple macérât huileux. Ainsi, suivant l'invention on associe les avantages des huiles végétales et de molécules hydrophiles (flavonoïdes, composés phénoliques). Enfin, le véhicule huileux évite de devoir ajouter un conservateur à la composition, celui-ci permettant en effet de stabiliser dans le temps les propriétés anti-oxydantes des extraits lipophiles de rose de porcelaine et de jacinthe d'eau et donc l'efficacité des compositions cosmétiques la renfermant. De plus, la formulation sous forme de composition huileuse permet une libération prolongée des molécules actives extraites de la rose de porcelaine et de la jacinthe d'eau. Ainsi, une seule application d'une composition cosmétique renfermant la composition huileuse de l'invention a une action efficace et durable sur l'éclat de la peau pendant une période d'environ 48 heures.

La composition cosmétique à application topique selon l'invention est caractérisée en ce qu'elle comprend, à titre d'ingrédient, au moins une composition huileuse conforme à l'invention et telle que définie précédemment.

La teneur en composition huileuse dans la composition cosmétique peut varier généralement de 0,01 à 50 % en masse, de préférence de 0,1 à 10% environ en masse, et encore plus préférentiellement de 0,5 à 4,0 % environ en masse, par rapport à la masse totale de la composition.

Le choix de la quantité de composition huileuse dans la composition peut être fait en fonction de l'utilisation envisagée. Pour un traitement prolongé, on utilise de préférence des doses plus faibles, sous forme de lait ou de crème dosée à environ 0,1 à 2 %, tandis qu'un traitement ponctuel peut nécessiter des doses plus élevées, par exemple un gel ou un sérum dosé entre 3 et 10 %.

Ainsi, ces compositions topiques peuvent être utilisées avantageusement en cosmétologie pour améliorer l'éclat de la peau, en particulier via sa couleur en augmentant la composante rose et en diminuant la composante jaune/verte.

Les compositions selon l'invention peuvent contenir, outre la composition huileuse conforme à l'invention, un ou plusieurs actifs secondaires renforçant ou complétant avantageusement son activité, et compatibles, c'est-à-dire non susceptibles de réagir les uns avec les autres ou de masquer ou limiter ses effets.

Plus particulièrement, les actifs secondaires peuvent être choisis parmi un agent anti-âge, ou parmi l'acétyl tetrapeptide-5 (CAS N° 820959-17-9), un extrait de plancton, un extrait de maca qui a un effet sur la prévention de dérèglements liés à une déficience de la microcirculation cutanée et au stress oxydatif qui peut en résulter, un extrait de pétales de coquelicot agissant sur la nutrition cellulaire, une combinaison d'extraits d'anchuse, de coquelicot et de passiflore qui, en association, ont un effet dans la prévention des signes du vieillissement cutané, un extrait de pancratium maritimum, un extrait de crocus décoloré, un extrait de graines de mimosa, un extrait de pétales de souci, des cellules de cacao, la vitamine C, la vitamine E et l'hexylrésorcinol.

L'agent anti-âge peut être par exemple un lipide tel que le géranyl géranyl isopropanol (Juvinity®) qui réduit la formation des peroxydes intracellulaires et retarde ainsi le vieillissement cellulaire.

Les compositions cosmétiques utilisables selon la présente invention possèdent une excellente tolérance cutanée, notamment en raison du film lipidique résultant du support huileux (huiles végétales, en particulier huile d'avocat et huile de rosier muscat), elles ne présentent aucune phototoxicité et leur application sur la peau, pour des périodes de temps prolongées, n'implique aucun effet systémique.

Les compositions utilisables selon la présente invention peuvent se présenter sous les formes galéniques classiquement utilisées pour une application topique, c'est-à-dire sous forme de gel, d'émulsion (en particulier crème ou lait), d'émulsion biphasique huile-dans-eau ou eau-dans-huile, d'huile corporelle, de masque, de pommade, d'onguent, de lotion, de solution concentrée, de nanocapsules, de liposomes, lesdites formes galéniques contenant en outre des excipients et supports usuels et acceptables sur le plan cosmétologique. Elles sont utilisées de préférence sous forme de crèmes, de sérums, de lotion ou de gel.

Ces formes d'administration par voie topique sont préparées par les techniques usuelles, et par exemple, dans le cas d'une crème, par dispersion d'une phase grasse dans une phase aqueuse pour obtenir une émulsion huile-dans-eau, ou inversement pour préparer une émulsion eau-dans-huile. Dans le cas de crèmes, on peut utiliser des émulsions à structure lamellaire obtenues avec des lécithines hydrogénées ou des sucro-esters, contenant peu de produits éthoxylés ou n'en contenant pas du tout.

Les compositions cosmétiques utilisables suivant l'invention peuvent aussi prendre la forme de lotion ou solution dans laquelle les extraits sont sous forme encapsulée dans des microsphères. Les microsphères suivant l'invention peuvent par exemple être constituées de corps gras, d'agar et d'eau. La composition huileuse utilisable conformément à l'invention peut être incorporée dans des vecteurs de type liposomes, glycosphères, dans des chylomicrons, des macro-, micro-, nano-particules ainsi que les macro-, micro- et nanocapsules et aussi être adsorbés sur des polymères organiques poudreux, les talcs, bentonites et autres supports minéraux.

Ces émulsions jouissent d'une bonne stabilité et peuvent être conservées pendant le temps nécessaire pour l'utilisation à des températures comprises entre 0 et 50°C sans qu'il y ait sédimentation des constituants ou séparation des phases.

Les compositions topiques utilisables selon l'invention peuvent comprendre des excipients appropriés pour une application topique externe, en particulier des excipients acceptables sur le plan cosmétologique. Ces excipients appropriés pour la formulation sont bien connus de l'homme du métier et comprennent en particulier des agents de pénétration tels que le phytantriol, l'octyl dodécanol et l'escine ; les épaississants tels que les gommes naturelles et les polymères de synthèse ; les tensioactifs ; les émollients tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsionnants tels que des dérivés de polyglycérol ; les conservateurs tels que le phénoxyéthanol et l'acide déhydroacétique ; les colorants ; les parfums ; etc...

Comme autres ingrédients utilisables dans les compositions de l'invention, on peut citer les agents hydratants tels que la glycérine, le butylène glycol, le sel de sodium de l'acide pyrrolidone carboxylique (sodium PCA), ainsi que des associations de dérivés glucosiques à effet hydratant et restructurant comme le produit Aquaxyl® (xylitylglucoside et anhydroxylytol et xylitol), et également les vitamines antioxydantes telles que la vitamine E, par exemple l'acétate de tocophérol ou le tocotriénol, la vitamine C, les polyphénols naturels.

On peut aussi ajouter à la composition des glucides choisis principalement parmi le glucose, le glycogène et le tréhalose, ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que d'une manière générale toute association avec un céramide.

On peut également ajouter à la composition des agents de protection contre les rayons ultraviolets, et par exemple des filtres solaires UV-A et UV-B hydrophiles ou lipophiles, ou des pigments formant écran anti-ultraviolet.

Les filtres solaires peuvent être choisis par exemple parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-β,β-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butyl-méthoxy dibenzoylméthane (Parsol 1789®), et le 4-méthoxy-dibenzoylméthane.

Les pigments peuvent être choisis par exemple parmi le dioxyde de titane, l'oxyde de zinc et l'oxyde de zirconium.

L'utilisation cosmétique de la composition cosmétique conforme à l'invention comprend tous les soins de la peau, en particulier les soins du visage, y compris les produits solaires, protecteurs et bronzants, les produits anti-âge, anti-séborrhéiques, toniques, les produits assurant l'amélioration de l'aspect de la peau y compris le traitement acnéique et les rougeurs cutanées.

Les exemples suivants illustrent l'invention plus en détail sans en limiter la portée. Dans tous les exemples de compositions qui suivent, les parties sont exprimées en masse, sauf indication contraire.

### EXEMPLES

### Exemple 1 : Préparation d'une huile contenant un extrait lipophile de jacinthe d'eau

On a utilisé des jacinthes d'eau (*Eichhornia crassipes*), plantes entières, préalablement broyées et séchées à froid. La technique du séchage à froid est préférable car elle permet de préserver les anti-oxydants présents dans la plante. Suivant cette technique, les plantes sont soumises à un premier broyage, puis à une déshydratation à température ambiante à l'abri de la lumière, de manière à ramener la teneur en eau vers 10%, et broyées à froid, à une température de -80°C, au moyen d'un broyeur à couteaux.

La teneur en eau était de 7,0% après broyage et séchage à froid.

1 kg de jacinthe d'eau ainsi séchée et broyée, sous forme de poudre fine et homogène, a été mélangé à 5 L d'huile vierge de jojoba. Le rapport plante / huile est d'environ 1/8. Le mélange a été maintenu sous flux d'azote pendant 4 heures à température ambiante.

Le mélange a ensuite été chauffé rapidement par micro-ondes (1.000 watts, 2 x 3 min.), sous flux d'azote, la température maximale atteinte étant de 150°C.

Le mélange a ensuite été traité par ultrasons (20 kHz), sous azote et sous agitation, pendant 10 minutes. L'huile contenant l'extrait d'*Eichhornia crassipes* a été séparée par centrifugation.

### Exemple 2 : Mise en évidence de l'absence de cytotoxicité de l'huile contenant un extrait lipophile de jacinthe d'eau

Dans cet exemple on a testé la cytotoxicité éventuelle de l'huile telle qu'obtenue par le procédé de l'Exemple 1.

Le test sur la viabilité d'épidermes humains reconstruits utilisé est celui de la réduction au bleu de Formazan (test MTT). L'essai a été réalisé *in vitro* sur épidermes humains reconstruits (« *Reconstructed Human Epidermis* », RHE) modèle RHEps, de surface 0,5 cm², vendus par la société SkinEthic ®.

L'huile préparée à l'exemple 1 a été testée pur (100 %), en application topique sur les épidermes.

Les mesures de viabilité tissulaires par test MTT ont été effectués après 24 heures d'incubation à 37°C en atmosphère humide air-CO₂ (35%/5%), un épiderme non traité servant de témoin.

Les résultats sont reportés dans le tableau 2 ci-après.

**Tableau 2**

| Substance | Densité optique DO 540 nm | % |
|---|---|---|
| Lot n°1 (témoin) | 0,398 ± 0,05 | - |
| Lot n°2 (huile pure) | 0,437 ± 0,05 | ns |

Aucune cytotoxicité n'est observée par rapport au témoin.

### EXEMPLE 3 : Préparation d'une huile comprenant un extrait lipophile de rose de porcelaine

Une huile comprenant un extrait lipophile d'*Etlingera elatior* a été préparée comme indiqué ci-après.

On a utilisé les fleurs et les tiges de la plante, préalablement séchée, puis broyée à froid.

L'extraction a été réalisée selon le procédé général décrit ci-dessus à l'exemple 1, mais en utilisant 1 kg d'*Etlingera elatior* séchée et broyée, sous forme de poudre fine et homogène, et 5 kg d'huile de colza.

### EXEMPLE 4 : Mise en évidence des propriétés de la composition huileuse sur l'amélioration de l'éclat de la peau

Dans cet exemple, on a étudié l'effet d'une composition huileuse conforme à l'invention sur l'éclat de la peau.

### 6.1. Principe du test

L'évaluation de l'éclat de la peau est basée sur la méthode « C.L.B.T. » qui est une analyse sensorielle visuelle par un jury composés de 3 juges auxquels on demandé de noter les critères suivants sur une échelle d'évaluation : la couleur de la peau (« *Colors* »), sa luminosité (« *Luminosity* »), son éclat (« *Brightness* ») et sa transparence (« *Transparency* »).

Les juges ont été éduqués durant une séance d'entrainement selon la méthodologie décrite par Musnier C et al. (« Visual evaluation in vivo of complexion radiance using the CLBT ™ sensory methodology », Skin Research and Technology, 2004, 10, 50-56).

Selon cette méthode, les critères d'évaluation sont définis de la façon suivante :
- Les Couleurs de la peau : il s'agit des pigments contenus dans la peau. Les couleurs sont le «vert olive», le «jaune» et le « rose ». Celles-ci sont utilisées pour décrire les différentes nuances du teint de la peau :
   * le « vert olive » : l'optimum à atteindre est l'absence de « vert olive » ;
   * le «jaune»: variation attendue: diminution de la composante « jaune » ;
   * le « rose » : variation attendue : augmentation de la composante « rose », l'optimum à atteindre étant la composante rose maximale (saturation).
- La luminosité : elle correspond à l'intensité de taches de lumière sur les zones saillantes du visage, l'optimum à atteindre étant une luminosité maximale.
- L'éclat : il s'agit d'une synthèse de l'uniformité de la couleur de la peau et de la régularité de la texture de la peau (homogénéité de la peau), l'optimum à atteindre étant un éclat maximal.
- La transparence : elle correspond à la caractéristique de la peau suivant laquelle il est possible ou non de voir les veines, reflétant ainsi la finesse de l'épaisseur de la peau, l'optimum à atteindre étant une transparence maximale.

### 6.2. Protocole du test

L'évaluation est réalisée dans une pièce noire sont l'atmosphère est contrôlée en température et en humidité relative (température 22°C ± 2°C ; humidité relative : 50 % ± 10 %).

Les sujets à évaluer subissent tout d'abord une période d'acclimatation de 20 minutes dans la pièce avant l'évaluation proprement dite.

L'étude a été effectuée sur un panel de 20 volontaires.

Les sujets sont assis entre deux lampes symétriques simulant la lumière du jour. Ils portent une charlotte noire sur la tête afin de cacher entièrement les cheveux ainsi qu'un peignoir noir sur les épaules afin d'éviter toute influence éventuelle d'une quelconque couleur extrinsèque.

L'évaluation est réalisée sur l'ensemble de la peau du visage.

Une pré-évaluation est d'abord réalisée afin de vérifier que les volontaires répondent aux critères d'inclusion dans l'étude sensorielle. Cette pré-évaluation est réalisée par un technicien qui vérifie si le volontaire a le teint terne ou non et si sa peau présente ou non des tâches pigmentaires.

Chacun des volontaires est évalué individuellement par trois juges différents qui ne peuvent pas communiquer entre eux.

L'évaluation des couleurs de la peau est effectuée en référence à un nuancier présentant chacune la gamme des trois couleurs. Pour chaque couleur, la saturation est graduée de 10 à 100 %, le 100% correspondant à une couleur complètement saturée. Chaque évalue chaque couleur individuellement et lui attribue un grade de saturation entre 10 et 100 %.

L'évaluation des trois autres critères (L., B. et T.) est effectuée individuellement pas chacun des 3 juges en utilisant une échelle analogique allant de 0 à 10 pour chacun des critères :
* L : 0 = aucune luminosité ; 10 = luminosité maximale ;
* B : 0 = aucun éclat ; 10 = éclat maximal ;
* T : 0 = aucune transparence ; 10 = transparence maximale.

Pour chacun des critères L., B. et T. évalués, une moyenne des évaluations effectuées par les trois juges est calculée.

En ce qui concerne les couleurs, les résultats sont exprimés en pourcentage de saturation.

L'interprétation des résultats est effectuée à partir des variations des différents critères d'évaluation de la façon suivante :
* une augmentation de la couleur « rose » est significative d'une amélioration de l'éclat de la peau, celle-ci paraissant globalement plus saine, c'est-à-dire plus jeune, plus fraîche et plus reposée ;
* une diminution de la couleur « jaune » et de la couleur « vert olive » est significative d'une amélioration de l'éclat du teint ; on dit que la peau « retrouve sa santé » ;
* une augmentation de la luminosité du teint montre que la peau reflète la lumière de façon plus intense ;
* une augmentation de l'éclat dénote un teint ayant une apparence améliorée en termes de texture et de couleur ;
* une augmentation de la transparence est significative d'une peau plus fine.

### 6.3. Compositions testées / Conditions d'application

Une composition de type « émulsion éclaircissante » conforme à l'invention, c'est-à-dire renfermant une composition huileuse constituée à 90 % en volume de l'huile comprenant un extrait de rose de porcelaine et telle que préparée ci-dessus à l'exemple 3 et 10 % en volume de l'huile comprenant un extrait de jacinthe d'eau et telle que préparée ci-dessus à l'exemple 1 a été évaluée.

La formulation de cette composition est donnée ci-après :

| | |
|---|---|
| - EDTA tetrasodique | 0,1 g |
| - Composition huileuse de l'invention | 0,5 g |
| - Vitamine C | 1,0 g |
| - Glycérine | 3,0 g |
| - Extrait de *Pancratium maritimum* | 0,5 g |
| - Extrait décoloré de *Crocus sativum* | 0,3 g |
| - Carbomer vendu sous la dénomination Carbopol® Ultrez 10 par la société Gattefossé | 0,2 g |
| - Mélange d'alcool arachidylique, d'alcool Behénique et de glucoside d'arachidyle vendu sous la dénomination Montanov® 202 par SEPPIC | 3,0 g |
| - Oléate de décyle | 5,0 g |
| - Huile d'amande douce | 3,0 g |
| - Vitamine E | 0,5 g |
| - Eau | qsp 100,0 g |

Bien entendu, la formulation de cette composition n'était pas mentionnée sur les emballages et n'a pas été communiquée aux membres du panel de volontaires.

Conditions d'application : cette composition a été appliquée 2 fois par jour de façon standard sur le visage pendant 1 mois par chacun des 20 membres du panel.

### 6.4. Résultats

Les résultats d'évaluation C.L.B.T sont reportés dans le tableau après :

**TABLEAU 4**

| **Critère d'évaluation** | **Score obtenu** |
|---|---|
| L : Luminosité | 15,2 % |
| B : Eclat | 20,7 % |
| T : Transparence | 18,3 % |
| Couleur Vert olive | -26,8 % |
| Couleur jaune | - 19,2 % |
| Couleur rose | +22,6% |

Ces résultats montrent que la composition testée renfermant la composition huileuse conforme à l'invention entraine une diminution significative des composantes « vert olive » et «jaune », et une augmentation de la composante « rose ». Ces résultats démontrent que l'utilisation d'une composition huileuse conforme à l'invention permet d'améliorer l'éclat de la peau.

De plus, ces essais ont montré que ces extraits n'ont aucun effet irritant ou allergisant pour la peau.

### EXEMPLE 5 : Emulsion pour améliorer l'éclat de la peau (émulsion « éclaircissante »)

Par les techniques usuelles, on prépare une émulsion ayant la composition massique indiquée ci-après.

| | |
|---|---|
| - EDTA tetrasodique | 0,1 g |
| - Glycol | 3,0 g |
| - Carbomer vendu sous la dénomination Carbopol® Ultrez 10 par la société Gattefossé | 0,2 g |
| - Mélange d'alcool arachidylique, d'alcool Behénique et de glucoside d'arachidyle vendu sous la dénomination Montanov® 202 par SEPPIC | 3,0 g |
| - Oléate de décyle | 5,0 g |
| - Huile d'amande douce | 3,0 g |
| - Acide citrique | 0,1 g |
| - Système de conservation | 0,8 g |
| - Composition huileuse de l'ex. 3/Composition huileuse de l'ex. 1 (90/10 ; v/v) | 1,0 g |
| - Vitamine C | 1,0 g |
| - Extrait de crocus blanc décoloré | 1,0 g |
| - Parfum | 0,5 g |
| - Eau | qsp 100,0 g |

Cette émulsion peut être utilisée une à deux fois par jour par application sur les zones de la peau à traiter.

### EXEMPLE 6: Sérum pour améliorer l'éclat de la peau (sérum « éclaircissant »)

Par les techniques usuelles, on a préparé sérum ayant la composition massique suivante :

| | |
|---|---|
| - EDTA tetrasodique | 0,1 g |
| - Glycol | 4,0 g |
| - Acrylate de sodium | 2,0 g |
| - Mélange d'alcool arachidylique, d'alcool behénique et de glucoside d'arachidyle vendu sous la dénomination Montanov® 202 par SEPPIC | 2,0 g |
| - Oléate de décyle | 3,0 g |
| - Huile de tournesol | 1,0 g |
| - Phényl triméthicone | 1,0 g |
| - Acide citrique | 0,1 g |
| - Système de conservation | 0,8 g |
| - Composition huileuse de l'ex. 3/Composition huileuse de l'ex. 1 (90/10 ; v/v) | 1,0 g |
| - Vitamine C | 2,0 g |
| - Extrait de crocus blanc décoloré | 2,0 g |
| - Parfum | 0,5 g |
| - Eau | qsp 100,0 g |

Ce sérum peut être utilisé une à deux fois par jour par application sur les zones de la peau à traiter.

### EXEMPLE 7 : Mousse nettoyante (éclat du visage)

Par les techniques usuelles, on a préparé une mousse nettoyante ayant la composition massique suivante :

| | |
|---|---|
| - EDTA tetrasodique | 0,1 g |
| - Glycérine | 3,0 g |
| - Polymère réticulé d'acrylate/C₁₀₋₃₀ alkyle acrylate vendu sous la dénomination commerciale Carbopol® Ultrez 21 par Gattefossé | 0,8 g |
| - NaOH | 0,3 g |
| - Lauroyl sarcosinate de sodium | 7,0 g |
| - Système de conservation | 0,8 g |
| - Composition huileuse de l'ex. 3/Composition huileuse de l'ex. 1 (90/10 ; v/v) | 1,0 g |
| - Vitamine C | 0,5 g |
| - Extrait de crocus blanc décoloré | 1,0 g |
| - Parfum | 0,5 g |
| - Eau | qsp 100,0 g |

Cette mousse nettoyante peut être appliquée une à deux fois par jour pour nettoyer et améliorer l'éclat de la peau du visage.

### EXEMPLE 8 : Masque pour le visage

Par les techniques usuelles, on a préparé un masque pour le visage ayant la composition massique suivante :

| | |
|---|---|
| - EDTA tetrasodique | 0,1 g |
| - Glycol | 3,0 g |
| - Carbomer vendu sous la dénomination Carbopol® | 0,2 g |
| - Mélange d'alcool arachidylique, d'alcool behénique et de glucoside d'arachidyle vendu sous la dénomination Montanov® 202 par SEPPIC | 3,0 g |
| - Oléate de décyle | 5,0 g |
| - Huile d'amande douce | 2,0 g |
| - Acide citrique | 0,1 g |
| - Système de conservation | 0,8 g |
| - Composition huileuse de l'ex. 3/Composition huileuse de l'ex. 1 (90/10 ; v/v) | 1,0 g |
| - Vitamine C | 1,0 g |
| - Extrait de crocus blanc décoloré | 0,3 g |
| - Parfum | 0,5 g |
| - Eau | qsp 100,0 g |

Ce masque peut être appliqué une à deux fois par semaine pour nettoyer et améliorer l'éclat de la peau du visage.

## Revendications

1. Composition huileuse, **caractérisée en ce qu'**elle comprend :
i) au moins une huile végétale V1 renfermant au moins un extrait lipophile de rose de porcelaine (*Etlingera elatior*) et
ii) au moins une huile végétale V2, renfermant au moins un extrait lipophile de jacinthe d'eau (*Eichhornia crassipes*) ;
lesdites huiles V1 et V2 (huiles vectrices) étant identiques ou différentes l'une de l'autre.

2. Composition selon la revendication 1, **caractérisée en ce que** :
- la quantité d'huile végétale V1 renfermant l'extrait lipophile de rose de porcelaine varie de 70 à 95 % en masse, et **en ce que**
- la quantité d'huile végétale V2 renfermant l'extrait lipophile de jacinthe d'eau varie de 30 à 5 % en masse,
lesdits pourcentages étant exprimés par rapport à la masse totale de la composition huileuse.

3. Composition selon la revendication 1 ou 2, **caractérisée en ce que** la composition huileuse comprend environ 90 % en masse d'huile végétale V1 renfermant l'extrait lipophile de rose de porcelaine et environ 10 % en masse d'huile végétale V2 renfermant l'extrait lipophile de jacinthe d'eau.

4. Composition selon l'une quelconque des revendications précédentes, **caractérisée en ce que** les huiles végétales V1 et V2, identiques ou différentes l'une de l'autre, sont choisies parmi l'huile de colza, l'huile de jojoba, l'huile d'avocat, l'huile de rosier muscat, l'huile de noisette, l'huile de macadamia, l'huile d'argan, l'huile de tournesol, l'huile de cameline, l'huile de bourrache, huile d'amande douce, huile de carthame, huile de calophyllum et leurs mélanges.

5. Composition selon l'une quelconque des revendications précédentes, **caractérisée en ce que** la composition huileuse comprend un mélange d'huile de colza (HC) et d'huile de jojoba (HJ) dans un rapport massique HC/HJ variant de 70/30 à 90/10.

6. Composition selon l'une quelconque des revendications précédentes, **caractérisée en ce que** la composition huileuse comprend 90 % en masse d'huile de colza renfermant au moins un extrait lipophile de rose de porcelaine et 10 % en masse d'huile de jojoba renfermant au moins un extrait lipophile de jacinthe d'eau.

7. Composition selon l'une quelconque des revendications précédentes, **caractérisée en ce que** lesdits extraits sont obtenus par extraction à l'abri de l'air.

8. Composition cosmétique à application topique, **caractérisée en ce qu'**elle comprend, à titre d'ingrédient, au moins une composition huileuse telle que définie à l'une quelconque des revendications 1 à 7.

9. Composition cosmétique selon la revendication 8, **caractérisée en ce que** la teneur en composition huileuse varie de 0,1 à 10% en masse par rapport à la masse totale de la composition.

10. Composition cosmétique selon la revendication 9, **caractérisée en ce que** la teneur en composition huileuse varie de 0,5 à 4,0 % en masse, par rapport à la masse totale de la composition.

11. Composition cosmétique selon l'une quelconque des revendications 8 à 10, **caractérisée en ce qu'**elle contient en outre un ou plusieurs actifs secondaires choisis parmi un agent anti-âge, l'acétyl tetrapeptide-5, un extrait de plancton, un extrait de maca, un extrait de pétales de coquelicot, une combinaison d'extraits d'anchuse, de coquelicot et de passiflore, un extrait de pancratium maritimum, un extrait de crocus décoloré, un extrait de graines de mimosa, un extrait de pétales de souci, des cellules de cacao, la vitamine C, la vitamine E et l'hexylrésorcinol.

12. Composition cosmétique selon l'une quelconque des revendications 8 à 11, **caractérisée en ce qu'**elle se présente sous forme de gel, d'émulsion, d'émulsion biphasique huile-dans-eau ou eau-dans-huile, d'huile corporelle, de masque, de pommade, d'onguent, de lotion, de solution concentrée, de nanocapsules ou de liposomes.

13. Composition cosmétique selon l'une quelconque des revendications 8 à 12, **caractérisée en ce qu'**elle comprend en outre des agents de protection contre les rayons ultraviolets ou des pigments formant écran anti-ultraviolet.

14. Procédé cosmétique non thérapeutique pour améliorer l'éclat de la peau, consistant à appliquer sur les zones de la peau concernées au moins une composition cosmétique telle que définie à l'une quelconque des revendications 8 à 13.

15. Utilisation non thérapeutique d'une composition cosmétique à application topique comprenant une composition huileuse telle que définie à l'une quelconque des revendications 1 à 7 pour améliorer l'éclat de la peau.

## Patentansprüche

1. Ölige Zusammensetzung, **dadurch gekennzeichnet, dass** sie umfasst:
i) mindestens ein Pflanzenöl V1, das mindestens einen lipophilen Extrakt von Fackelingwer *(Etlingera eliator)* einschließt, und
ii) mindestens ein Pflanzenöl V2, das mindestens einen lipophilen Extrakt von Wasserhyazinthe *(Eichhornia crassipes)* einschließt,
wobei die Öle V1 und V2 (Vektoröle) identisch oder unterschiedlich voneinander sind.

2. Zusammensetzung nach Anspruch 1, **dadurch gekennzeichnet, dass**:
- die Menge des Pflanzenöls V1, das den lipophilen Extrakt von Fackelingwer einschließt, von 70 bis 95 Ma% schwankt, und dass
- die Menge des Pflanzenöls V2, das den lipophilen Extrakt von Wasserhyazinthe einschließt, von 30 bis 5 Ma% schwankt,
wobei diese Prozentsätze in Bezug zur Gesamtmasse der öligen Zusammensetzung ausgedrückt sind.

3. Zusammensetzung nach Anspruch 1 oder 2, **dadurch gekennzeichnet, dass** die ölige Zusammensetzung zirka 90 Ma% Pflanzenöl V1, das den lipophilen Extrakt von Fackelingwer einschließt, und zirka 10 Ma% Pflanzenöl V2, das den lipophilen Extrakt von Wasserhyazinthe einschließt, umfasst.

4. Zusammensetzung nach einem der vorangehenden Ansprüche, **dadurch gekennzeichnet, dass** die Pflanzenöle V1 und V2, identisch oder unterschiedlich voneinander, aus dem Rapsöl, dem Jojobalö, dem Avocadoöl, dem Weinrosenöl, dem Haselnussöl, dem Macadamiaöl, dem Arganöl, dem Sonnenblumenöl, dem Leindotteröl, dem Borretschöl, dem Mandelöl, dem Färberdistelöl, dem Calophyllumöl und ihren Gemischen ausgewählt sind.

5. Zusammensetzung nach einem der vorangehenden Ansprüche, **dadurch gekennzeichnet, dass** die ölige Zusammensetzung ein Gemisch aus Rapsöl (HC) und Jojobaöl (HJ) in einem Massenverhältnis HC/HJ umfasst, das von 70/30 bis 90/10 schwankt.

6. Zusammensetzung nach einem der vorangehenden Ansprüche, **dadurch gekennzeichnet, dass** die ölige Zusammensetzung 90 Ma% Rapsöl, das mindestens einen lipophilen Extrakt von Fackelingwer einschließt, und 10 Ma% Jojobaöl, das mindestens einen lipophilen Extrakt von Wasserhyazinthe einschließt, umfasst.

7. Zusammensetzung nach einem der vorangehenden Ansprüche, **dadurch gekennzeichnet, dass** die Extrakte durch Extraktion unter Luftabschluss gewonnen sind.

8. Kosmetische Zusammensetzung zur topischen Anwendung, **dadurch gekennzeichnet, dass** sie als Inhaltsstoff mindestens eine ölige Zusammensetzung nach einem der Ansprüche 1 bis 7 umfasst.

9. Kosmetische Zusammensetzung nach Anspruch 8, **dadurch gekennzeichnet, dass** der Gehalt an öliger Zusammensetzung von 0,1 bis 10 Ma% in Bezug zur Gesamtmasse der Zusammensetzung schwankt.

10. Kosmetische Zusammensetzung nach Anspruch 9, **dadurch gekennzeichnet, dass** der Gehalt an öliger Zusammensetzung von 0,5 bis 4,0 Ma% in Bezug zur Gesamtmasse der Zusammensetzung schwankt.

11. Kosmetische Zusammensetzung nach einem der Ansprüche 8 bis 10, **dadurch gekennzeichnet, dass** sie einen oder mehrere sekundäre Wirkstoffe enthält, ausgewählt aus einem Anti-Age-Wirkstoff, dem Acetyltetrapeptid-5, einem Planktonextrakt, einem Macaextrakt, einem Extrakt aus Blütenblättern von Klatschmohn, einer Kombination aus Extrakten von Ochsenzunge, Klatschmohn und Passionsblume, einem Extrakt von Dünen-Trichternarzisse, einem Extrakt aus entfärbtem Krokus, einem Extrakt aus Mimosensamen, einem Extrakt aus Blütenblättern von Ringelblume, Kakaozellen, Vitamin C, Vitamin E und Hexylresorcinol.

12. Kosmetische Zusammensetzung nach einem der Ansprüche 8 bis 11, **dadurch gekennzeichnet, dass** sie in Form von Gel, von Emulsion, von Zweiphasen-Öl-in-Wasser- oder -Wasser-in-Öl-Emulsion, von Körperöl, von Maske, von Pomade, von Salbe, von Lotion, von konzentrierter Lösung, von Nanokapseln oder von Liposomen auftritt.

13. Kosmetische Zusammensetzung nach einem der Ansprüche 8 bis 12, **dadurch gekennzeichnet, dass** sie ferner Schutzmittel gegen ultraviolette Strahlen oder Pigmente umfasst, die einen Schirm gegen ultraviolette Strahlen bilden, umfasst.

14. Nicht therapeutisches kosmetisches Verfahren zur Verbesserung des Strahlens der Haut, das darin besteht, auf die betroffenen Zonen der Haut mindestens eine kosmetische Zusammensetzung nach einem der Ansprüche 8 bis 13 aufzutragen.

15. Nicht therapeutische Verwendung einer kosmetischen Zusammensetzung zur topischen Anwendung, umfassend eine ölige Zusammensetzung nach einem der Ansprüche 1 bis 7 zur Verbesserung des Strahlens der Haut.

## Claims

1. Oily composition, **characterized in that** it comprises:
i) at least one vegetable oil VI containing at least a lipophilic extract of torch ginger (*Etlingera elatior*); and
ii) at least one vegetable oil V2 containing at least a lipophilic extract of water hyacinth (*Eichhornia crassipes*);
said oils V1 and V2 (vector oils) being the same or different.

2. The composition according to claim 1, **characterized in that**:
- the amount of vegetable oil V1 containing the lipophilic extract of torch ginger varies from 70 to 95 % by weight, and **in that**
- the amount of vegetable oil V2 containing the lipophilic extract of water hyacinth varies from 30 to 5 % by weight,
said percentages being expressed relative to the total weight of the oily composition.

3. The composition according to claim 1 or 2, **characterized in that** the oily composition comprises about 90 % by weight of vegetable oil V1 containing the lipophilic extract of torch ginger and about 10 % by weight of vegetable oil V2 containing the lipophilic extract of water hyacinth.

4. The composition according to any of the preceding claims, **characterized in that** the vegetable oils V1 and V2, the same or different, are selected from among rapeseed oil, jojoba oil, avocado oil, rose hip oil, hazelnut oil, macadamia oil, argan oil, sunflower seed oil, camelina oil, borage oil, sweet almond oil, safflower oil, tamanu oil and mixtures thereof.

5. The composition according to any of the preceding claims, **characterized in that** the oily composition comprises a mixture of rapeseed oil (HC) and jojoba oil (HJ) in a weight ratio HC/HJ varying from 70:30 to 90:10.

6. The composition according to any of the preceding claims, **characterized in that** the oily composition comprises 90 % by weight of rapeseed oil containing at least a lipophilic extract of torch ginger and 10 % by weight of jojoba oil containing at least a lipophilic extract of water hyacinth.

7. The composition according to any of the preceding claims, **characterized in that** said extracts are obtained by airtight extraction.

8. Cosmetic composition for topical application, **characterized in that**, as ingredient, at it comprises at least one oily composition such as defined in any of claims 1 to 7.

9. The cosmetic composition according to claim 8, **characterized in that** the oily composition content varies from 0.1 to 10 % by weight relative to the total weight of the composition.

10. The cosmetic composition according to claim 9, **characterized in that** the oily composition content varies from 0.5 to 4.0 % by weight relative to the total weight of the composition.

11. The cosmetic composition according to any of claims 8 to 10, **characterized in that** it further comprises one or more secondary active ingredients selected from among an anti-ageing agent, acetyl-tetrapeptide-5, a plankton extract, maca extract, poppy petal extract, combination of extracts of Anchusa, poppy and Passiflora, an extract of *pancratium maritimum,* extract of *crocus discolor*, extract of mimosa seeds, extract of marigold petals, cocoa cells, vitamin C, vitamin E and hexylresorcinol,

12. The cosmetic composition according to any of claims 8 to 11, **characterized in that** it is in the form of a gel, emulsion, biphasic oil-in-water or water-in-oil emulsion, body oil, mask, ointment, unguent, lotion, concentrated solution, nanocapsules or liposomes.

13. The cosmetic composition according to any of claims 8 to 12, **characterized in that** it further comprises agents protecting against ultraviolet radiation or pigments forming an anti-ultraviolet screen.

14. Non-therapeutic cosmetic method to improve skin radiance, consisting of applying to the skin regions concerned at least one cosmetic composition such as defined in any of claims 8 to 13.

15. Non-therapeutic use of a cosmetic composition for topical application comprising an oily composition such as defined in any of claims 1 to 7 to improve skin radiance.
